# FASCICULE DE BREVET EUROPEEN

(11) **EP 0 521 752 B1**
(45) Date de publication et mention de la délivrance du brevet: **22.03.1995**
(21) Numéro de dépôt: 92401744.5
(22) Date de dépôt: 22.06.1992
(51) Int. Cl.: C07C 67/343, C07C 69/653

(54) **Procédé de préparation d'acrylates alpha fluorés**
Verfahren zum Herstellen von Alpha-fluorierten Acrylaten
Process for the preparation of alpha fluorinated acrylates

(30) Priorité: 02.07.1991 FR 9108220
(43) Date de publication de la demande: 07.01.1993
(73) Titulaire: ELF ATOCHEM S.A., 92800 Puteaux (FR)
(72) Inventeur: Grison, Claude, F-54500 Vandoeuvre Les Nancy (FR); Boulliung, Nathalie, F-54000 Nancy (FR); Coutrot, Philippe, F-54420 Saulxures Les Nancy (FR)
(74) Mandataire: Rieux, Michel

(56) Documents cités:
- BULLETIN DE LA SOCIETE CHIMIQUE DE FRANCE. no. 3, 1985, PARIS FR pages 448 -454 G.ETEMAD-MOGHADAM 'Synthèse stéréosélective d'esters alpha,béta-éthyléniques alpha fluorés E par réaction de Wittig-Horner à partir du diéthylphosphono alpha-fluoroacétate de méthyle.Etude comparative avec le diphénylphosphonoxy alpha-fluoroacétate de méthyle.'

## Description

La présente invention se rapporte à un procédé de préparation d'acrylates α fluorés, elle a plus particulièrement pour objet la mise au point d'un nouveau procédé de préparation d'acrylates α fluorés permettant d'obtenir des produits fluorés de grande pureté avec des rendements élevés tout en mettant en oeuvre un mode de fabrication de réalisation facile et peu coûteux.

Les monomères acryliques et méthacryliques sont des composés qui ont été largement étudiés et développés dans l'industrie à cause de leur réactivité. Cette réactivité permet la mise en oeuvre de nombreuses réactions chimiques en particulier la réalisation de diverses réactions de polymérisation et de copolymérisation conduisant à des matériaux dont les applications sont très variées. C'est ainsi par exemple que les polyméthacrylates fabriqués à partir de divers acrylates ou méthacrylates et notamment le polyméthacrylate de méthyle ou PMMA ainsi que certains matériaux fabriqués à partir de divers acrylates ou méthacrylates sont des produits particulièrement intéressants en raison de leurs propriétés optiques et mécaniques. On les utilise par exemple dans l'industrie automobile et dans l'industrie aéronautique pour la fabrication de pare-brise, de hublots et de cockpits.. Toutefois, leur faible tenue thermique et leur mauvaise résistance aux agents chimiques limitent dans une certaine mesure leur champ d'application par exemple, dans le domaine de l'aviation supersonique. Pour ces raisons depuis quelques années, de nouveaux produits acryliques ayant des structures chimiques particulières ont été développés.

En particulier, plusieurs dérivés fluorés acryliques, notamment des esters α-fluorés acryliques ont fait l'objet de nombreuses études. Ces esters α-fluorés acryliques répondent à la formule générale 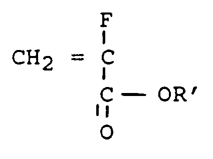
dans laquelle R′ est un radical alcoyle et le plus souvent un radical méthyle ou éthyle ou un radical aryle notamment un radical phényle.

C'est ainsi que l'art antérieur décrit l'obtention d'α-fluoroacrylates à partir de dihalogéno propanes dans une réaction en deux étapes comprenant un traitement par une mélange de HN0₃ et d'HF suivi d'une déshalogénation par le Zn en milieu sulfurique (demande européenne 415 214) ou par un traitement par le fluor de divers esters acryliques (demande européenne 383 128) ou à partir d'un halogénure de l'acide α-fluoroacrylique, T. Nguyen et C. WAKSELMAN, Journal Org. Chem 54, 5640 (1989) ou à partir de dérivés du type 2,2 difluoro 1- méthyle cyclopropane (demande européenne 398 061) ou à partir d' α-fluoroacroléine, H. MOLINES, T. NGUYEN et C. WAKSELMAN Synthesis, 755 (1985) ou par traitement avec le paraformaldéhyde de l'énolate d'un diéthyle fluoro oxalo acétate, E.D. BERGMANN et I SHAHAK, Journal Chem. Soc. 4033 (1961), ou en quatre étapes à partir de vinyle éthyle éther avec un rendement global de 40 %, T. NGUYEN, H. MOLINES et C. WAKSELMAN, Synthese Comm. 15,925 (1985) ou encore en trois étapes à partir de 2,2 3,3- tétrafluoro 1- propanol avec un rendement global de 40 %, A. THENAPPAN et D.3. BURTON, Tetr. Letters 30, 5571 (1989).

L'art antérieur décrit également la préparation de l'α-fluoro acrylate d'éthyle à partir d'α-fluoro phosphono acétate d'éthyle et de formiate d'éthyle par action conjuguée du butyl lithium et de l'hydrure de diisobutyle aluminium dans l'éther à basses températures, A. THENAPPAN et D.3. BURTON, Journal of Fluorine Chemistry, 48, 153 (1990) et Journal Org. Chem. 55, 4639 (1990) ainsi que la synthèse de divers α-fluoroacrylates substitués répondant à la formule générale XYC = CF-COOR dans laquelle CXY est différent de CH₂, par condensation d'un α-fluoro phosphono acétate avec divers aldéhydes ou cétones soit en présence de naH dans l'éther diéthylique à basses températures H. MACHLEIDT et R. WESSENDORF, Ann der Chem. 674,1 (1964) ; E. ELKIK et Ch. FRANCESCH, Bull. Soc. Chim. France, 5, 783 (1985) soit par action de Butyl lithium dans le THF à - 70°C, G.E. MOGMADAM et J. SEYDENPENNE, Bull. Soc. Chim. France, 3, 448 (1985) ; Ph. COUTROT et C. GRISON, Journal Organomet. Chem. 1, 332 (1987).

Tous ces procédés présentent à des degrés divers le désavantage soit de nécessiter la mise en oeuvre des réactifs coûteux ou de manipulation délicate, soit d'opérer dans les conditions difficilement extrapolables au niveau industriel, soit de conduire à des rendements médiocres ne dépassant pas 60 % dans le meilleur des cas.

Le besoin se fait donc sentir de disposer de procédés permettant la mise en oeuvre de réactifs de manipulation aisée et peu- coûteux en vue d'obtenir des esters α fluorés acryliques de bonne qualité avec de bons rendements.

La présente invention concerne un procédé de fabrication d'acrylates α fluorés de formule générale I : 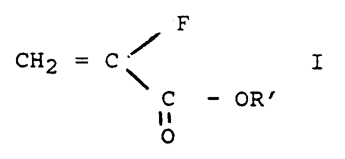
dans laquelle R′ est un radical alcoyle linéaire ou ramifié ayant de 1 à 12 atomes de carbone, ou un radical cycloalkyle mono ou polycondensé ayant de 3 à 10 atomes de carbone, un radical aryle ayant de 6 à 10 atomes de carbone, non substitués ou substitués par un ou plusieurs groupements alcoyle et comprenant éventuellement un ou plusieurs hétéroatomes, tels que S, N, O ou P. selon lequel le procédé comporte deux étapes : la synthèse d'un α-fluoro phosphono acétate de façon connue dans une première étape, l'obtention de l'acrylate α fluoré préparé dans la seconde étape par condensation de l'α-fluoro-phosphono acétate avec un aldéhyde caractérisé en ce que l'aldéhyde utilisé est le formaldéhyde que l'on met en oeuvre en l'état ou sous forme de paraformaldéhyde la réaction de condensation étant réalisée en milieu aqueux en présence d'une base faible minérale.

On a trouvé que la mise en oeuvre d'un tel procédé permettait d'opérer dans des conditions relativement douces et conduisait aux acrylates de formule générale I avec de bons rendements. De plus, les conditions opératoires mises en oeuvre selon le procédé de l'invention ne nécessitent pas l'utilisation de réactifs organo-métalliques coûteux qui nécessitent par ailleurs, des opérations de récupération contraignantes.

Une des formes particulières du procédé de la présente invention consiste à utiliser comme source de formaldéhyde du paraformaldéhyde en solution aqueuse. Selon l'invention le paraformaldéhyde est dépolymérisé dans le milieu réactionnel,par tous moyen connu de l'homme de l'art par exemple en présence d'acide phosphorique au moment de la réaction de condensation avec l'α fluoro phosphono acétate. On a en effet trouvé que la dépolymérisation du paraformaldéhyde au moment de l'emploi permettait de réaliser ensuite une bonne condensation avec le l'α-fluoro-phosphono acétate. Selon l'invention on met en oeuvre 0,33 à 10 moles de paraformaldéhyde pour 1 mole α-fluoro-phosphono acétate.

Selon un autre caractéristique du procédé de l'invention la condensation du formaldéhyde avec l'α-fluoro phosphono acétate est réalisée en présence d'une base faible. Selon l'invention la base est choisie de préférence parmi un sel d'acide minéral faible : en particulier sels de l'acide carbonique, le métal étant un métal alcalin ou alcalino-terreux. Selon l'invention on utilise de préférence le carbonate de potassium. La quantité du sel d'acide faible utilisée est comprise entre 1 et 5 moles pour 1 mole de l'α-fluoro-phosphonoacétate.

Selon l'invention on fabrique de façon connue dans une première étape par une réaction dite d'ARBUSOV un α-fluoro phosphono acétate. Un α-fluoro-diéthyle phosphono acétate d'éthyle tel que II peut être obtenu par exemple par réaction de bromofluoroacétate d'éthyle sur le triéthylphosphonate selon la réaction : 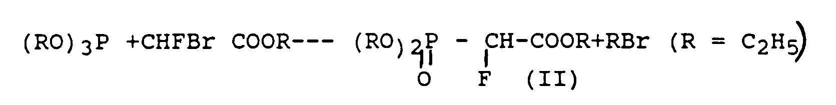
Il est possible également de façon connue de préparer divers α-fluoro phosphono acétates d'alcoyle par transestérification d'un α-fluoro phosphono acétate tel que II précédemment décrit, par divers alcools en présence de catalyseurs tels que par exemple un tétra alcanoate de titane Ti(OR)₄. Il est aussi possible, de façon connue d'obtenir des α-fluoro phosphono acétates d'aryle à partir d'un acide dialkylphosphono-2 fluoroacétique. Ce dernier est transformé en chlorure d'acide qui est ensuite soumis à une réaction avec un phénol en présence d'une base telle que la pyridine ou une amine tertiaire ou d'un alcoolate alcalin.

Les exemples suivants illustrent la présente invention.

Toutes les quantités sont exprimées en parties en poids.

### EXEMPLE 1

### a/ Préparation du diéthylphosphono-2 fluoro acétate d'éthyle (II) :

Dans un réacteur comportant une colonne à distiller et muni d'un dispositif de chauffage, on introduit 10 parties de bromofluoroacétate d'éthyle que l'on préchauffe sous agitation à 70°C. On ajoute ensuite 9,8 parties de triéthylphosphate par petites quantités de façon à ce que le bromure d'éthyle formé distille au fur et à mesure du degré d'avancement de la réaction. La température du milieu réactionnel est amenée progressivement à 140°C et on maintient l'agitation à cette température pendant 4 heures. Après refroidissement du milieu réactionnel on recueille 11,13 parties de phosphonate (II) par distillation sous vide. Teb_{O,6} mm : 112°, Rdt : 85 %
Le spectre de RMN de ce produit est conforme à la structure attendue.

### b/ Préparation de l'α-fluoroacrylate d'éthyle

Dans un réacteur muni d'un agitateur et d'un dispositif de chauffage, on introduit :
3,35 parties de paraformaldéhyde
O,126 parties d'une solution d'acide phosphorique 1N.
2,5 parties d'eau.

Le mélange est chauffé à 90°C pendant 1 heure 30 minutes ce qui conduit à une solution claire de formaldéhyde. On ajoute ensuite à cette solution 2,23 parties de diéthylphosphono -2 fluoro - 2 acétate d'éthyle. Le mélange réactionnel est agité à température ambiante pendant 30 minutes. On ajoute ensuite goutte à goutte une solution de carbonate de potassium constituée de 10 millimoles de carbonate de potassium soit 1,4 parties dans 3 parties d'eau. L'addition de cette solution alcaline provoque une montée en température du milieu réactionnel qui passe de 20°C à 40°C. On agite ensuite le mélange à cette température pendant 15 minutes puis on le ramène à la température ambiante à l'aide d'un bain d'eau glacée. On ajoute ensuite au milieu réactionnel 6 parties d'éther et 4 parties d'une solution aqueuse saturée en chlorure de sodium. Après extraction à l'éther la phase organique est séchée sur sulfate de magnésium. Après évaporation du solvant on recueille 0,9 parties de α-fluoroacrylate d'éthyle ce qui correspond à un rendement de 82 %. Les spectres infra-rouge et de résonance magnétique nucléaire permettent de confirmer la structure du produit obtenu. 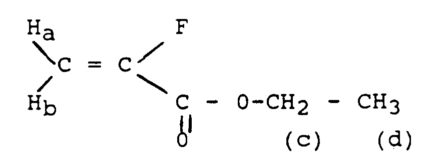
- SPectre Infra-Rouge : 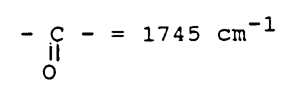 〉C = C〈 1650 cm ⁻¹
- Spectre R.M.N. :
   Déplacement chimique (δ)
   (CH3)_{(d)} : 1.36 ppm
   (CH2)_{(c)} : 4,31 ppm
   (CH)₍ₐ₎ : 5,29 ppm
   Constante de couplage (J)
   (Hₐ - F) : 13 Hertz
   (Hₐ - H_{b}) : 1,5 Hertz
   (H_{b} - F) : 42 Hertz
   (H_{b} - Ha) : 1,5 Hertz

### EXEMPLE 2

### a/ Préparation du diisopropylphosphono-2 fluoro-2 acétate de n-butyle

On prépare selon le mode opératoire décrit dans l'exemple 1 (a) le diisopropylphosphono-2 fluoro-2 acétate de méthyle.

Ce produit est ensuite soumis à une réaction de transestérification à l'aide de n-butanol. Dans un réacteur muni d'un dispositif de chauffage, d'une agitation et d'un appareillage de reflux, on introduit 1,49 parties de diisopropylphosphono 2 fluoro-2 acétate de méthyle, 0,43 parties de n-butanol, 0,22 parties de tétrabutanonate de titane et 30 parties de benzène. Le mélange est porté à reflux pendant 24 heures. Après évaporation du benzène on recueille 1,55 parties de diisopropylphosphono-2 fluoro-2 acétate de butyle ce qui correspond à un rendement de 90 %. Les spectres R.M.N. et infra-rouge permettent de confirmer la structure du produit obtenu qui a la structure suivante :

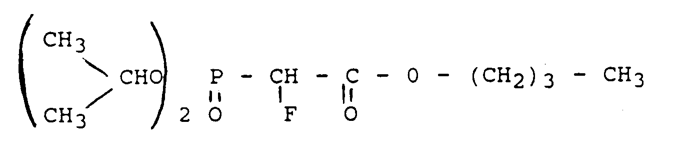

### b/ Préparation de l'α-fluoroacrylate de butyle

On prépare cet ester en mettant en oeuvre le même protocole opératoire que celui décrit dans l'exemple 1 (b) mais en partant de diisopropylphosphono-2 fluoro-2 acétate de n-butyle. Après traitement on recueille l'α-fluoroacrylate de n-butyle avec un rendement de 80 %.
Les spectres infra-rouge et de R.M.N. permettent de confirmer la structure de l'ester obtenu : 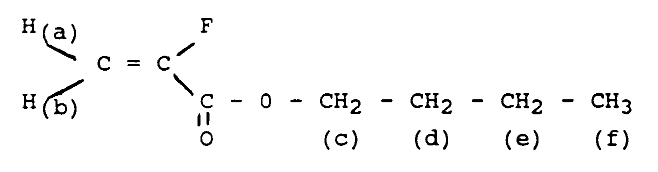
- Spectre infra-rouge (V) 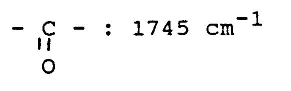 〉C = C〈: 1650 cm⁻¹
- Spectre R.M.N.
   Déplacement chimique (δ)
   (CH₃) : 0,95 ppm
   (CH₂)ₑ : 1,30 - 1,51 ppm
   (CH₂)_{d} : 1,71 ppm
   (CH₂)_{c} : 4,23 ppm
   (H)ₐ : 5,30 ppm
   Constante de couplage (J)
   (Hₐ - F) : 12,5 Hertz
   (Hₐ - H_{b}) : 1,5 Hertz
   (H_{b} - F) : 43 Hertz

### EXEMPLE 3

On répète le mode opératoire de l'exemple 1^{b} mais en partant du diisopropylphosphono-2 fluoro-2 acétate de méthyle comme produit intermédiaire de départ.

On recueille l'α-fluoroacrylate de méthyle avec un rendement de 70 %. Les spectres infra-rouge et de R.M.N. permettent de confirmer la structure du produit 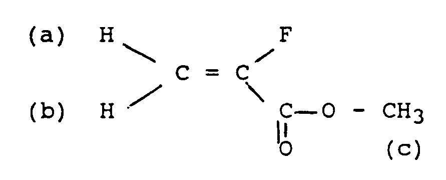
- Spectre infra-rouge
   (C = O) : 1745 cm⁻¹
   (C = C) : 1650 cm⁻¹
- Spectre R.M.N.
   Déplacement chimique (δ)
   (CH₃) : 3,85 ppm
   (H)ₐ : 5,35 ppm
- Constante de couplage (J)
   J Hₐ - F : 12,5 Hertz
   J Hₐ - H_{b} : 1,5 Hertz
   J H_{b} - F : 43 Hertz
   J H_{b} - Hₐ : 1,5 Hertz

## Revendications

1. Procédé de fabrication d'acrylates α fluorés de formule générale I : 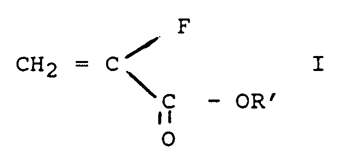 dans laquelle R' est un radical acoyle linéaire ou ramifié aynt de 1 à 12 atomes de carbone, un radical cycloalkyle mono ou polycondensé ayant de 3 à 10 atomes de carbone, ou un radical aryle ayant de 6 à 10 atomes de carbone, non substitués ou substitués par un ou plusieurs groupements alcoyle et comprenant éventuellement un ou plusieurs hétéroatomes tels que S, N, O ou P selon lequel le procédé comporte deux étapes : la synthèse d'un α-fluoro phosphono acétate de façon connue dans une première étape, l'obtention de l'acrylate α fluoré préparé dans la seconde étape par condensation de l'α-fluoro phosphono acétate avec un aldéhyde caractérisé en ce que l'aldéhyde utilisé est le formaldéhyde que l'on met en oeuvre en l'état ou sous forme de paraformaldéhyde la réaction de condensation étant réalisée en milieu aqueux en présence d'une base faible minérale.

2. Procédé selon 1, caractérisé en ce que le paraformaldéhyde est dépolymérisé dans le milieu réactionnel au moment de la réaction de condensation avec l'α-fluoro phosphono acétate.

3. Procédé selon 1 et 2 caractérisé en ce que la réaction de condensation est réalisée en présence d'une base faible choisie parmi un sel d'acide minéral faible.

4. Procédé selon 1 à 3 caractérisé en ce que le sel est du carbonate de potassium.

## Claims

1. Process for the manufacture of α-fluoroacrylates of general formula I: 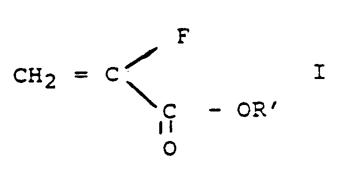 in which R' is a linear or branched alkyl radical containing from 1 to 12 carbon atoms, a mono- or polycondensed cycloalkyl radical containing from 3 to 10 carbon atoms, or an aryl radical containing from 6 to 10 carbon atoms, unsubstituted or substituted by one or more alkyl groups and optionally containing one or more heteroatoms such as S, N, O or P, according to which the process comprises two stages: the synthesis of an α-fluorophosphonoacetate in a known manner in a first stage and the obtaining of the α-fluoroacrylate prepared in the second stage by condensation of the α-fluorophosphonoacetate with an aldehyde, characterized in that the aldehyde employed is formaldehyde and that it is used as such or in the form of paraformaldehyde, the condensation reaction being carried out in an aqueous medium in the presence of a weak inorganic base.

2. Process according to 1, characterized in that paraformaldehyde is depolymerized in the reaction mixture at the time of the condensation reaction with the α-fluorophosphonoacetate.

3. Process according to 1 and 2, characterized in that the condensation reaction is carried out in the presence of a weak base chosen from a salt of a weak inorganic acid.

4. Process according to 1 to 3, characterized in that the salt is potassium carbonate.

## Patentansprüche

1. Verfahren zur Herstellung von α-fluorierten Acrylaten der allgemeinen Formel I 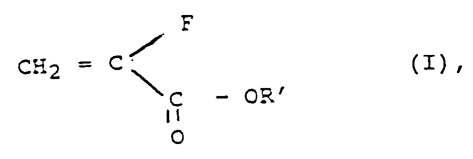 in der R' eine geradkettige oder verzweigte Alkylgruppe mit 1 bis 12 Kohlenstoffatomen, eine mono- oder polykondensierte Cycloalkylgruppe mit 3 bis 10 Kohlenstoffatomen oder eine Arylgruppe mit 6 bis 10 Kohlenstoffatomen darstellt, die ggfs. mit einer oder mehreren Alkylgruppen substituiert ist und ggfs. ein oder mehrere Heteroatome, wie z.B. S, N, O oder P, enthält, wobei das Verfahren zwei Schritte umfaßt:
- in einem ersten Schritt die Synthese eines α-Fluorphosphonoacetats in bekannter Weise,
- im zweiten Schritt die Herstellung eines α-fluorierten Acrylats durch Kondensation des α-Fluorphosphonoacetats mit einem Aldehyd,
dadurch gekennzeichnet,
daß der verwendete Aldehyd Formaldehyd ist, der in Form von Paraformaldehyd eingesetzt wird, wobei die Kondensationsreaktion in wäßrigem Medium in Gegenwart einer schwachen anorganischen Base durchgeführt wird.

2. Verfahren nach Anspruch 1, dadurch gekennzeichnet, daß das Paraformaldehyd in dem Reaktionsmedium im Moment der Kondensationsreaktion mit α-Fluorphosphonoacetat depolymerisiert wird.

3. Verfahren nach Anspruch 1 und 2, dadurch gekennzeichnet, daS die Kondensationsreaktion in Gegenwart einer schwachen Base durchgeführt wird, die unter den Salzen einer schwachen anorganischen Säure ausgewählt ist.

4. Verfahren nach einem der Ansprüche 1 bis 3, dadurch gekennzeichnet, daß das Salz Kaliumcarbonat ist.
